# EUROPEAN PATENT SPECIFICATION

(11) **EP 2 898 226 B1**
(45) Date of publication and mention of the grant of the patent: **06.09.2017**
(21) Application number: 13801705.8
(22) Date of filing: 19.09.2013
(51) Int. Cl.: F16B 2/06, F16B 7/04

(54) **FAST LOCKING DEVICE FOR AT LEAST ONE TOOL ON AT LEAST ONE GUIDE ELEMENT**
SCHNELLSPERRVORRICHTUNG FÜR MINDESTENS EIN WERKZEUG AUF MINDESTENS EINEM FÜHRUNGSELEMENT
DISPOSITIF DE VERROUILLAGE RAPIDE POUR AU MOINS UN OUTIL SUR AU MOINS UN ÉLÉMENT DE GUIDAGE

(30) Priority: 21.09.2012 IT BO20120509
(43) Date of publication of application: 29.07.2015
(73) Proprietor: MEKNA S.r.l., 38068 Rovereto (TN) (IT)
(72) Inventor: MERLO, Paolo, Rovereto (TN) (IT); ROSSI, Christian, Rovereto (TN) (IT)
(74) Representative: Nemni, Raffaelo
(86) International application number: PCT/IB2013/002055
(87) International publication number: WO 2014/045100

(56) References cited:
- EP-A2- 1 647 237
- US-A1- 2002 061 225
- US-A1- 2006 290 076

## Description

The present invention relates to the sector concerning tools for operating theatres and in particular it refers to a fast locking device for at least one tool on at least one guide element.

In the sector of tools for operating theatres, devices are known to fix tools and/or instruments to special guides locked to the operating table in correspondence with the edges or appendices of the operating plane. Such guides feature a rectangular transversal section and dimensions determined by international regulations to standardize the types of locking devices connectable thereto.

The simplest locking devices known so far comprise a pair of reciprocally and integrally locked clamps, the former of which is designed for being fixed to a guide of the operating table and the second is suitable for locking a tool. These clamps are usually driven by separate adjustment elements, for instance consisting of their respective "T"-head screws and engaging appropriate nut screws of the clamp. The two clamps are realized in one body, consequently they feature a fixed angle between the planes of action of the two clamps.

An evolution of said clamps known so far concerns the possibility of modifying the angle between the planes of action of the two clamps, in that the latter are implemented by two separate elements and are coupled to each other by a revolving and removable locking element which allows a mutual rotation of the two clamps as well as the possibility of fixing them in the desired position. The revolving locking element consists of a revolving pin and a third screw which, coupled with a nut screw present on at least one of the two clamps, acts onto the pin to prevent its rotation in the fixed condition.

A further evolution of the devices known so far concerns the replacement of the screw locking elements with snap-in and/or spring locking elements or a combination of the different types mentioned above.

A drawback of the fast locking systems for tools known so far concerns the presence of many locking elements to adjust and/or lock each individual movement of the tool or of the locking device on the guide of the operating table, which often requires the use of both operator's hands to perform the operation.

A further drawback concerns the high number of movable, hence subjected to wear and tear, elements and possible malfunctions present in the devices known so far which render the implementation and the subsequent use of the locking device complex and expensive. The relevant prior art to be considered is also described in the document US 2006/290076 A1 regarding a surgical tool holder that includes a holding member with a clamping jaw, which is movable between open and clamping positions. A gripping member is configured to supportingly grip a surgical tool and is selectively placed in a rotatable or fixed association with the holding member. Engagement portions of the holding member and gripping member are engagable to block rotation there between to obtain the fixed association. Another relevant document is US 2002/061225 A1 regarding a socket and rail clamp apparatus for securing a surgical accessory to a rail of a patient support device includes a first jaw and a second jaw coupled to the first jaw. One of the first and second jaws is movable between a first position in which the socket and rail clamp assembly is attachable to and detachable from the rail and a second position in which the clamp grips the rail. A socket assembly includes among a plurality of components a socket adapted to receive a portion of a surgical accessory. The plurality of components are movable between a loosened configuration and a tightened configuration.

The main purpose of the present invention is to provide a fast locking device for at least one tool that allows to fix said device onto a support guide, to adjust the position of the device and of the tool locked thereto by operating with one hand onto one adjustment element so as to make its use simpler.

A further purpose is to provide a device that comprises a limited number of movable elements in order to reduce both the implementation costs and the maintenance costs as well as to reduce the possible malfunctions.

Another purpose is to provide a device that offers more possibilities of use and adjustments with respect to the locking devices known so far while retaining a structure that is compact and does not hamper any operations performed around there.

The characteristics of the invention are highlighted below with a special reference to the attached drawings, in which:
- figure 1 shows an axonometric view of the fast locking device for tools according to the present invention in an operating condition whereby a tool is fixed to a guide;
- figure 2 shows a side view of the device according to the present invention alone;
- figure 3 shows a front view of the device shown in figure 2;
- figure 4A shows a cross-section side view according to plane IV-IV in figure 3 in a release condition A;
- figure 4B shows a cross-section side view according to plane IV-IV in figure 3 in a guide locked condition B;
- figure 4C shows a partial cross-section side view of the device shown in figure 3 according to plane IV-IV in a third condition;
- figure 5 shows a partial cross-section view according to plane V-V in figure 2;
- figure 6 shows a bottom view of a first element of the device shown in figure 2;
- figure 7 shows a cross-section view according to plane VII-VII in figure 6;
- figure 8 shows a bottom view of a second element of the device shown in figure 2;
- figure 9 shows a cross-section view according to plane IX-IX in figure 8;
- figure 10 shows an axonometric view of a third element of the device shown in figure 2;
- figure 11 shows a cross-section view according to plane XI-XI in figure 10;
- figure 12 shows an axonometric view of a fourth element of the device shown in figure 2.

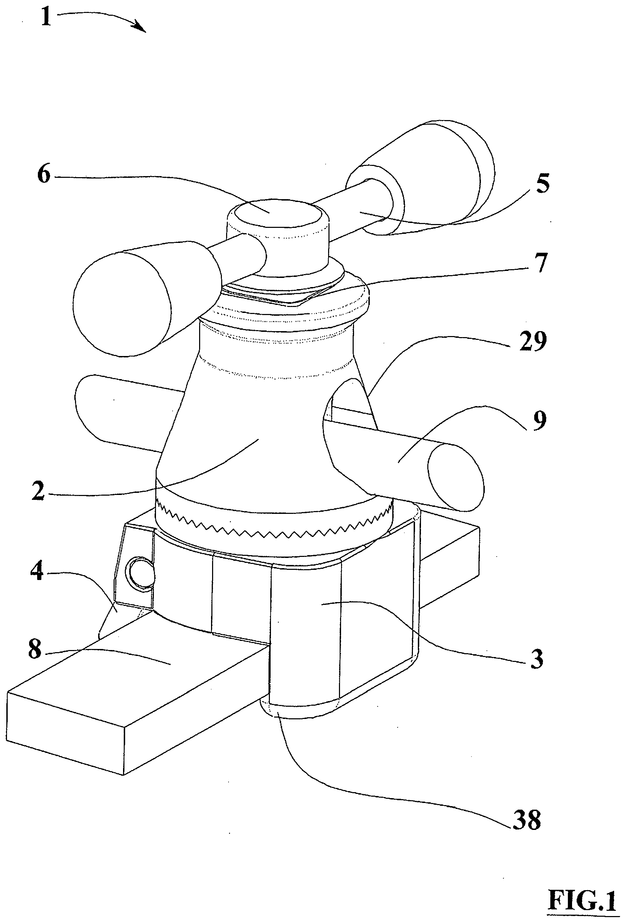

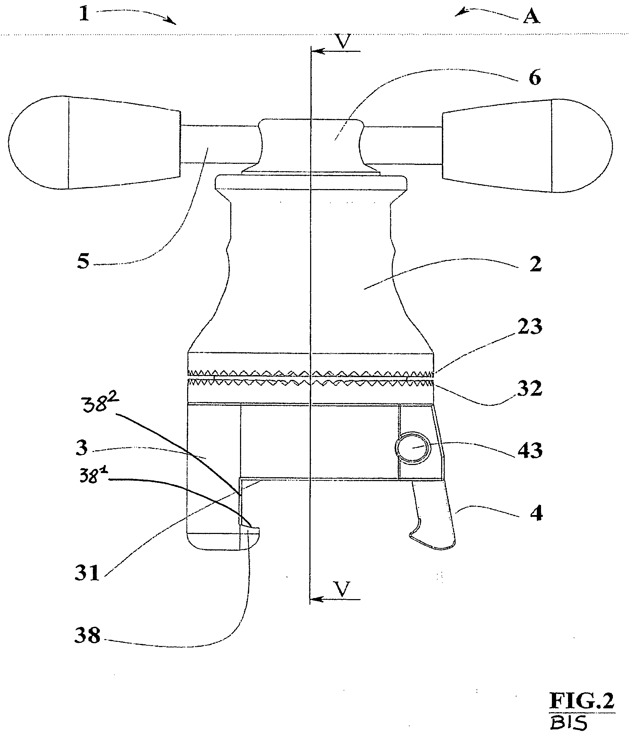

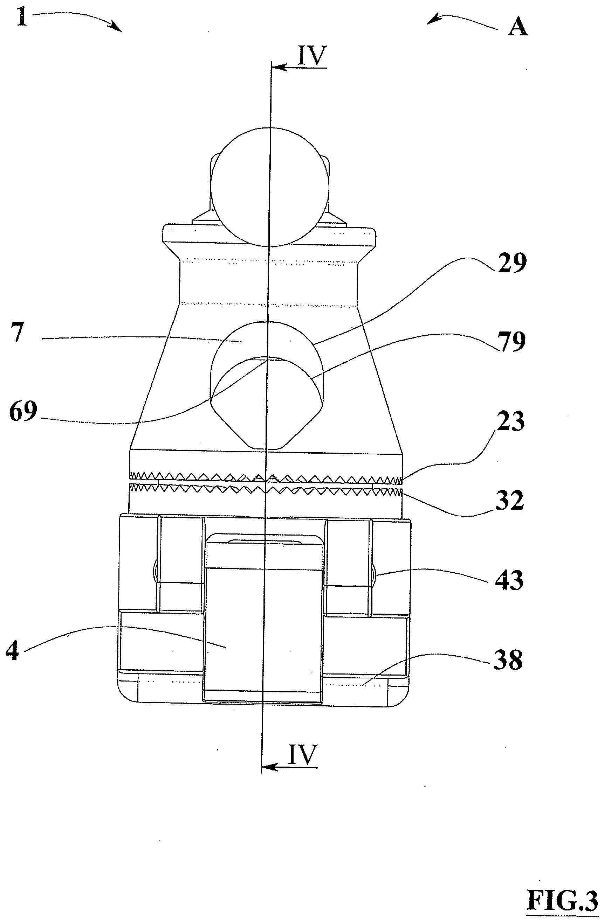

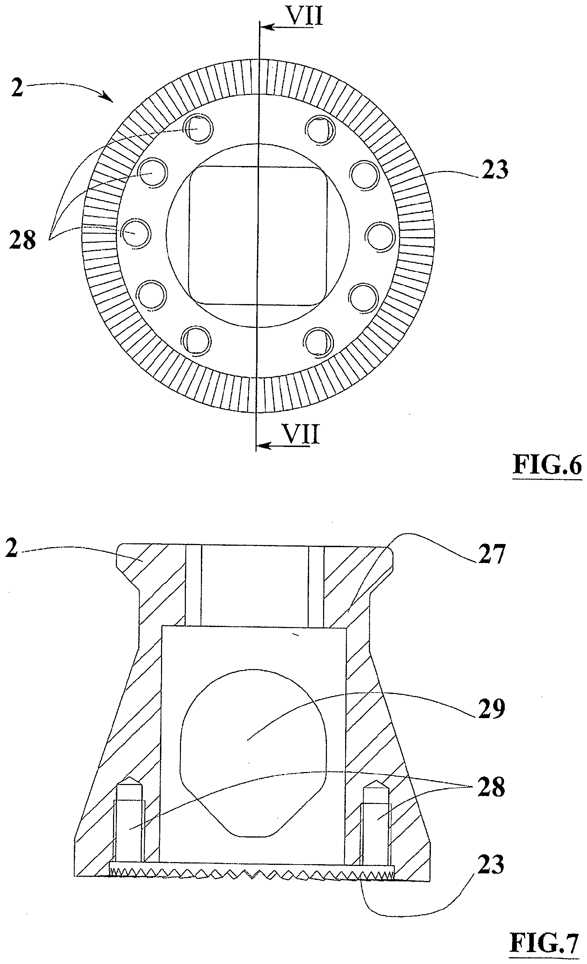

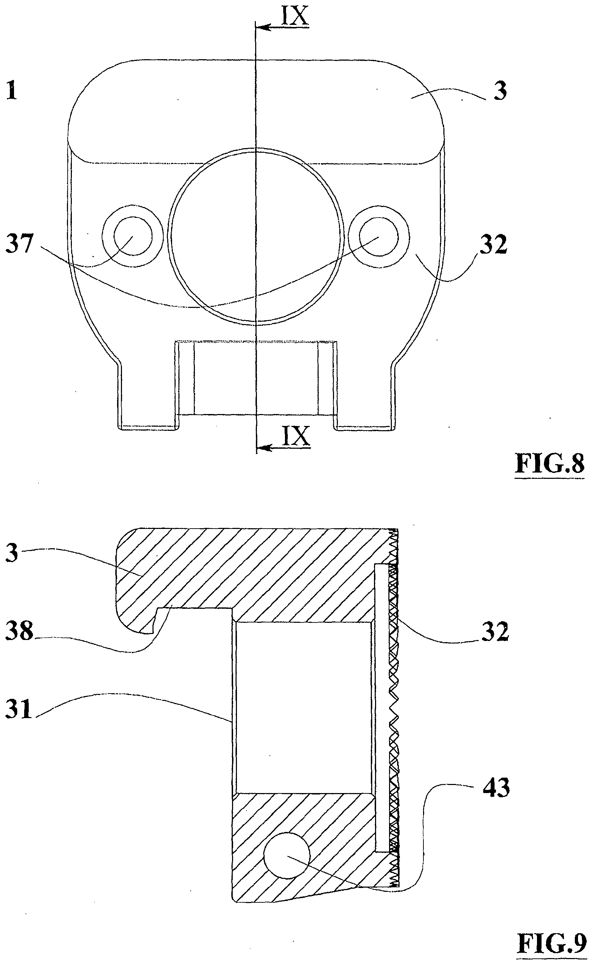

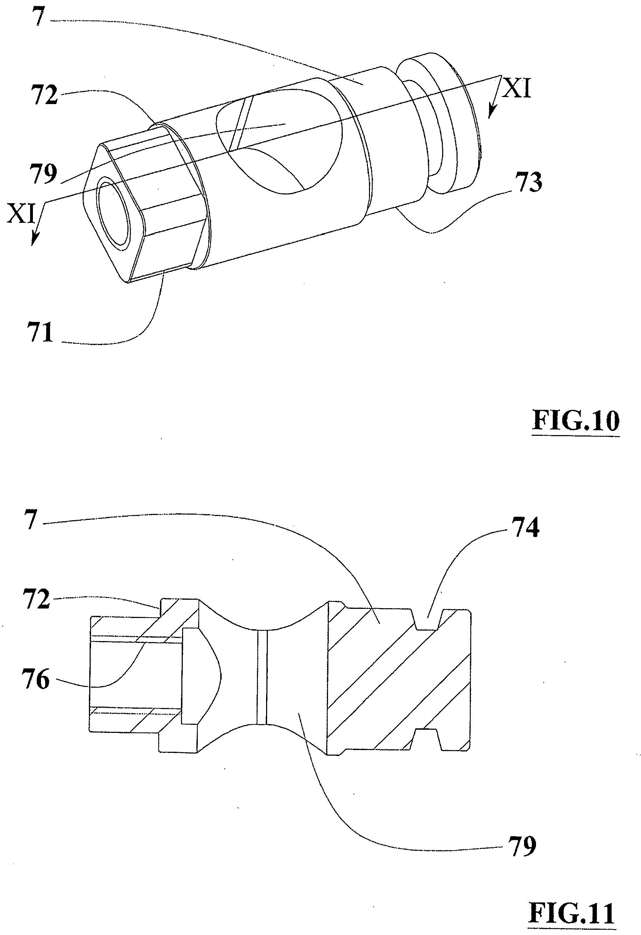

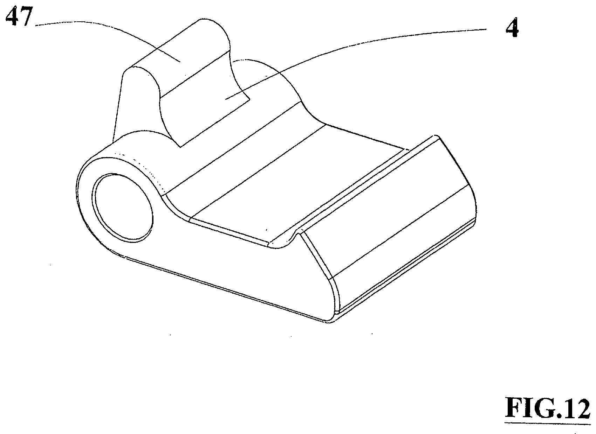

With reference to figures 1 thru 12, the numeral 1 identifies the fast locking device for at least one tool 9 on at least one guide element 8 according to the present invention.

In the preferred embodiment, the device 1 is used to fix tools having at least one elongated element featuring a standardized circular cross-section to guide elements featuring a standardized rectangular cross-section present at the sides of operating tables.

The device 1 comprises a fixing element 2 whose external shape is a truncated cone, provided with at least one first opening 29, which allows a tool to pass through, and diametrically crosses the fixing element 2 in correspondence with the tapered section of the truncated cone and develops parallel to the base surface of the truncated cone. The fixing element 2 also presents a variable-section transition which joins the two faces of the truncated cone.

A base 3 is removably lockable to the fixing element 2, by means of clutch means, in correspondence with the base of the truncated cone and is provided with a stop surface 31 opposite to the surface the fixing element 2 is lockable to. At an end of the stop surface 31 there is an "L"-shaped locking element 38 the function of which is to partially engage the guide element 8.

The clutch means comprise a first locking surface 23 on the fixing element 2 and a second locking surface 32 on the base 3 both featuring a sequence of tooth-like projections featuring a radial development.

A hook-like element 4 is revolvingly locked to the base 3 via a pin 43 located at the end of the stop surface 31 opposite to that where the locking element 38 is located.

The hook-like element features a projection 47 close to the pin 43 and facing the inside of the device 1.

The hook-like element 4 is movable between a release position A, in which it forms an obtuse angle with the base surface, and a locked position B, in which it forms an acute angle with the base surface and in conjunction with the locking element 38 locks the guide element 8 against the base 3.

A cursor element 7 is coaxially sliding internally to the fixing element 2 and to the base 3 and is provided with a second opening 79, which is substantially in line with the first opening 29 of the fixing element 2 in an operating condition, and a hollow 74 which engages the projection 47 of the hook-like element 4 so as to make it rotate around the pin 43 whenever the cursor element 7 moves internally to the base 3 and the fixing element 2.

The cursor element 7 features a circular section 73 close to a portion thereof sliding inside the base 3, to make it possible a mutual rotation between the cursor element 7 and the base 3, and a section featuring a sequence of flat faces 71 joined to each other by curved surfaces in correspondence with a portion thereof sliding inside the fixing means 3, so as to prevent a mutual rotation thereof. The circular section 73 and the flat-faces section 71 are jointed to each other by a shoulder 72 which abuts a thrust surface 27 internal to the fixing element 2 and parallel to the first locking surface 23.

A locking means 6 internally engages the cursor element 7 and is provided with an inner end 69 that leads to inside the first opening 29 and the second opening 79 to abut the tool 9 whenever the latter is located inside them.

The locking means 6 is an at least partially threaded element 67 which engages a nut screw 76 inside the cursor element 7 and close to the outer free end it is equipped with a control element 5, consisting of a sliding rocker arm or an end lever. Also, the locking means 6 comprises, close to the inner end 69, a safety element 66 which engages a corresponding seat 77 present in the cursor element 7 to prevent a complete slip off thereof.

A number of elastic elements 39, consisting of helical springs, are interposed between the base 3 and the fixing element 2 so as to counter their mutual approach. In order to improve the operation of said elastic element 39, an annular shim 25 is interposed between the fixing element 2 and the base 3 and constitutes the thrust surface which the elastic elements 39, possibly provided with a sphere-like end to improve its smoothness, act onto.

In order to make it possible to fix tools having different cross-sections, the first opening 29 and the second opening 79 feature a tapered shape.

In a first variant of the preferred embodiment of the device 1, the fixing element 2 presents a plurality of threaded holes 28 that can be aligned to their respective holes 37 present on the base 3 and whose function is to removably lock the position between the fixing element 2 and the base element 3 at predetermined angles of rotation.

In order to make it easier the identification of the correct angle of rotation on the outer surfaces of the fixing element 2 and of the base 3, there are reference indicators which show the angle of rotation with respect to an initial position.

In a second variant of the preferred embodiment of the device 1, the locking means 5 is provided with audible, tactile, or visual indicators to indicate the different operating conditions of the device 1 to the operator.

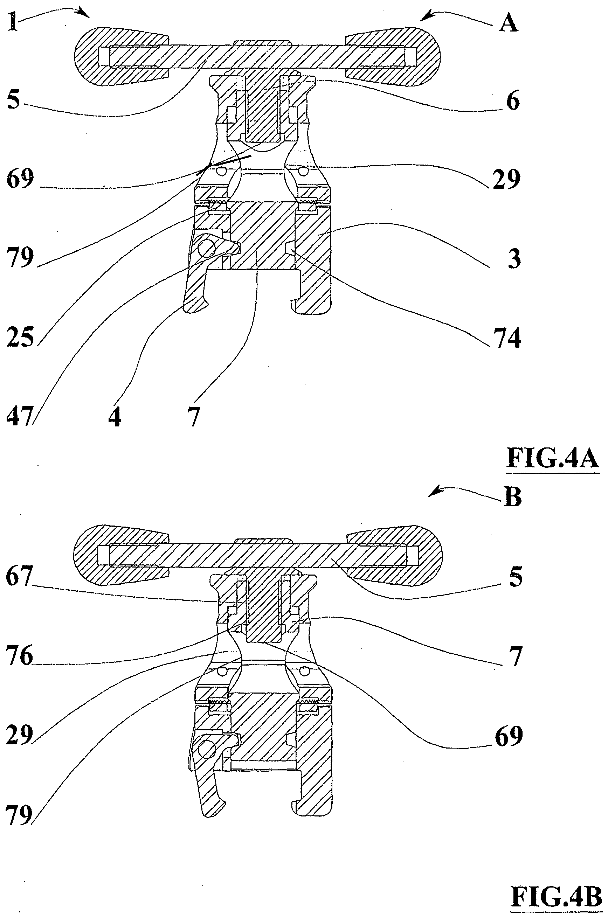

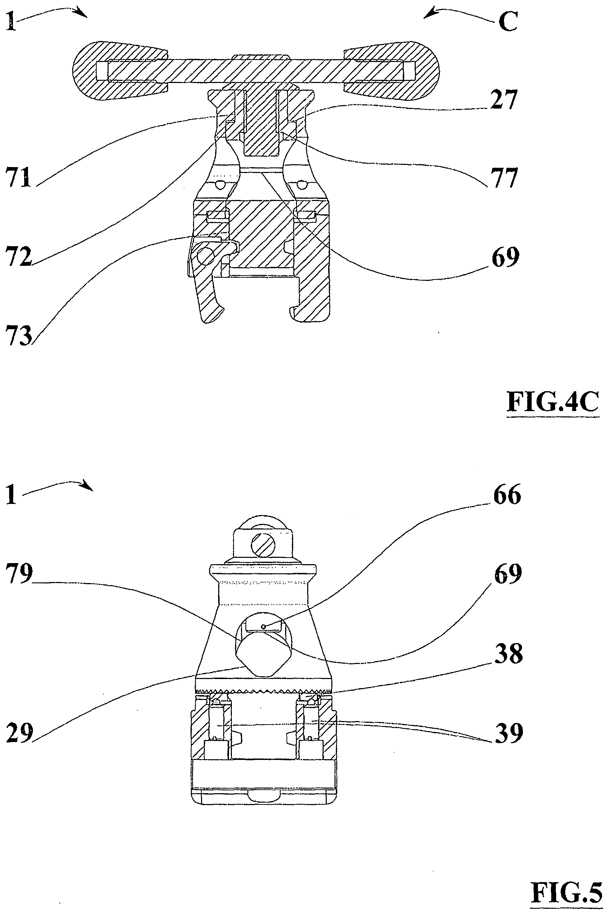

The device 1 supports the following operating conditions:
- release condition A, illustrated in figure 4A, in which the device 1 is not locked to the guide element 8 nor does it lock the tool 9, and the hook-like means 4 features an obtuse angle with respect to the stop surface 31;
- guide locked condition B, illustrated in figure 4B, in which the inner end 69 of the locking means 6 abuts the tool 9, and exerts a limited pressure onto the latter, and makes the cursor element 7 move and drives in its own movement the hook-like element from the release condition A to the locked position B in which it approaches the guide element 8 and locks it against the base 3, in this condition the fixing element 2 being able to rotate with respect to the base 3 and the tool 9 being able to move;
- rotation locking condition C, illustrated in figure 4C, in which because of the increased pressure exerted by the locking means 6 onto the tool 9, the cursor element 7 overcomes the resistance offered by the elastic element 38 and makes the fixing element 2 approach the base 3 up to mutually locking them, in such condition the tool 9 being free to move, such movement being however partially countered by the elastic element 38;
- tool locking condition, illustrated in figure 1, in which the locking means 7 is squeezed and the inner end 69 exerts such a force onto the tool 9 as to lock it.

In the operation of the preferred embodiment, the operator shall place the device 1, set to its release condition A, onto the guide 8 it shall be fixed to and makes it move along the latter up to the correct position. Having reached the correct position, the operator shall insert the tool 9 into the first opening 29 and the second opening 79, advantage being taken of the resistance offered by the elastic element 39 which limits the sliding capabilities of the tool.

Then the operator shall operate the locking means 6 by making the control element 5 rotate by approximately ¼ of turn clockwise, so as to make the cursor element 7 move, hence the hook-like element 4 rotate towards the base 3 up to locking the guide element 8 in correspondence with the guide locked conditions B.

In the guide locked condition B, the operator can vary the angle of rotation between the base 3 and the fixing element 2 at will to correctly position the tool as a function of the specific requirements of every operation.

Having reached the desired angle, the operator shall turn the control element 5 by a further ¼ of turn clockwise, which makes the cursor element 7 overcome the resistance of the elastic elements 38 and move further and approach the base 3 to the fixing element 2 up to locking them in the desired position, in correspondence with the rotation locking condition C.

In this condition the operator can easily move the tool 9 with respect to the device 1 up to reaching the optimum position for the operation.

Finally the operator shall turn the control element 5 by a further ¼ of turn clockwise to lock the tool 9, in correspondence with the tool locked condition.

Should it be necessary to vary the position of the tool 9 with respect to the device 1, the operator shall turn the control element 5 by approximately ¼ of turn counterclockwise so as to relieve the pressure of the locking means 6 onto the tool 9 and to make its movement possible.

Similar considerations apply, should it be necessary to vary the rotation between the base means 3 and the fixing means 2.

The main advantage of the present invention consists in that it provides a fast locking device for at least one tool that allows to fix said device onto a support guide, to adjust the position of the device and of the tool locked thereto by operating by one hand onto one adjustment element so as to make its use simpler.

A further advantage is in that it provides a device that features a limited number of movable elements so as to reduce the implementation costs and the maintenance costs as well as to reduce its possible malfunctions.

Another advantage is in that it provides a device that offers greater possibilities of use and a greater number of adjustment facilities as referred to the locking devices known so far, while retaining a structure that is compact and doesn't hamper the operations taking place in its vicinity, if any.

## Claims

1. A fast locking device for at least one tool (9) onto at least one guide element (8) suitable for adjusting the position of said device and at least one tool (9) by one hand onto the guide element (8) comprising:
- a fixing element (2) provided with at least one first opening (29) for the through-passage of at least one tool (9);
- a base (3) removably lockable to the fixing element (2), via clutch means, provided with a stop surface (31), opposite to the fixing element (2), at an end of which there is a locking element (38) to at least partially engage the guide element (8);
- a hook-like element (4) revolvingly locked to the base (3) via a pin (43) between a release condition (A) and a locking condition (B) for the guide element (8);
- a cursor element (7) sliding internally to the fixing element (2) and to the base (3) and provided with at least one second opening (79) and one hollow (74) which engages a projection (47) present on the hook-like element (4);
- a locking means (6) which internally engages the cursor element (7) and is provided with an inner end (69) that leads to the inside of the first opening (29) and the second opening (79) and in use abuts the tool (9);
- at least one elastic element (39) interposed between the base (3) and the fixing element (2) which counters their mutual approach;
said device supporting a release condition (A) in which the device (1) is free from the guide element (8) and from the tool (9);
said device being **characterized in that** it further supports:
- a guide locked condition (B) in which the base is locked to the guide element (8) by means of a first rotation of the locking means (6) driving the cursor element (7) which moves inside the base (3) engaging the protrusion (47) of the element of the hook (4) in such a way that said hook element (4) rotates around the pin (43) forming an obtuse angle with the stop surface (31) allowing to vary the angle of rotation between the base (3) and the fixing element (2) and to move the tool (9);
- a rotation locked condition (C) in which the rotation between the fixing element (2) and the base (3) is prevented by means of a second rotation in the same direction as the first one of the locking means (6) approaching the fixing element (2) to the base (3) until they engage each other, allowing to move the tool (9);
- a tool locked condition in which the tool (9) is locked to the fixing element (2) by means of a third rotation, in the same direction as the previous rotations of the locking means (6) tightening as pack the cursor (7) and the inner end (69) exerting on the tool (9) a force to lock it.

2. A device according to the previous claim, **characterized in that** the cursor element (7) features a circular section portion (73) slidable internally to said base (3) and a flat-faces section portion (71) slidable internally to the fixing element (2), said sections (73, 71) being joined by a shoulder (72) which abuts a thrust surface (27) internal to the fixing element (2).

3. A device according to any of the previous claims, **characterized in that** the locking means (6) comprises, close to the inner end (69), a safety element (66) which engages a corresponding seat (77) present in the cursor element (7) to prevent the complete slip off thereof.

4. A device according to any of the previous claims, **characterized in that** the first opening (29) and the second opening (79) feature a tapered shape to receive tools having different transversal sections.

5. A device according to claim 1 **characterized that** the locking element (38) is shaped as an "L" with the smallest portion of the inner face of the "L" (38.1) inclined so as to form an angle greater than 90 ° with the other inner face (38.2) of the"L"

## Patentansprüche

1. Schnell-Arretier-Vorrichtung für mindestens ein Werkzeug (9) auf mindestens einem Führungselement (8), dazu geeignet, die Position der genannten Vorrichtung und mindestens eines Werkzeugs (9) mit einer Hand am Führungselement (8) einzustellen, die Folgendes umfasst:
- ein Befestigungselement (2), ausgestattet mit mindestens einer ersten Öffnung (29) für den Durchtritt mindestens eines Werkzeugs (9);
- eine Basis (3), die an dem Befestigungselement (2), mit Kupplungsklammern, lösbar befestigbar ist, ausgestattet mit einer Anschlagfläche (31), dem Befestigungselement (2) gegenüberliegend, an deren einem Ende sich ein Feststellelement (38) befindet, um in das Führungselement (8) mindestens teilweise einzurasten;
- ein hakenähnliches Element (4), das an der Basis (3) mittels eins Stifts (43) drehbar befestigt ist
zwischen einem Lösezustand (A) und einem Arretierzustand (B) für das Führungselement (8);
- ein Schieberegler-Element (7), das sich im Innern von Befestigungselement (2) und Basis (3) verschiebt und ausgestattet mit mindestens einer zweiten Öffnung (79) und einer Vertiefung (74), in die eine, am hakenähnlichen Element (4) befindliche, Ausbuchtung (47) greift;
- ein Feststellmittel (6), das im Innern in das Schieberegler-Element (7) greift und mit einem inneren Ende (69) ausgestattet ist, das zum Innern der ersten Öffnung (29) und der zweiten Öffnung (79) führt und beim Gebrauch am Werkzeug (9) anliegt;
- mindestens ein elastisches Element, zwischengeschaltet zwischen der Basis (3) und dem Befestigungselement (2), das deren gegenseitiger Annäherung entgegenwirkt; wobei genannte Vorrichtung einen Lösezustand (A) unterstützt, in dem die Vorrichtung (1) vom Führungselement (8) und vom Werkzeug (9) frei ist;
wobei genannte Vorrichtung dahingehend gekennzeichnet ist, dass sie darüberhinaus unterstützt:
- einen Führungs-Arretierzustand (B), bei dem die Basis mit dem Führungselement (8) mittels einer ersten Drehung des Feststellmittels (6) verriegelt ist, wodurch das Schieberegler -Element (7) geführt wird, so dass es sich in die Basis (3) hinein bewegt und in die Ausbuchtung (47) des Hakenelements (4) greift, solchermaßen, dass sich das Hakenelement (4) rund um den Stift (43) dreht und dabei einen stumpfen Winkel mit der Anschlagfläche (31) bildet und es erlaubt, den Drehwinkel zwischen Basis (3) und Befestigungselement (2) zu verändern und das Werkzeug (9) zu bewegen;
- einen Drehungs-Arretierzustand (C), bei dem die Drehung zwischen Feststellelement (2) und Basis (3) mittels einer zweiten Drehung des Feststellmittels (6), in derselben Drehrichtung wie die erste, verhindert wird, wobei das Feststellelement (2) der Basis (3) angenähert wird, bis beide ineinandergreifen, und es möglich ist, das Werkzeug (9) zu bewegen;
- einen Werkzeug-Arretierzustand, bei dem das Werkzeug (9) mit dem Feststellelement (2) mittels einer dritten Drehung des Feststellmittels (6), in derselben Drehrichtung wie die vorherigen Drehungen, verriegelt ist, indem das Schieberegler-Element (7) und das innere Ende (69) als Block angezogen werden und auf das Werkzeug (9) eine Kraft ausüben, die es arretiert.

2. Ein Gerät nach dem vorhergehenden Anspruch, **dadurch gekennzeichnet, dass** das Schieberegler-Element (7) einen Teil mit rundem Querschnitt (73) aufweist, der innerhalb genannter Basis (3) verschiebbar ist, und einen Teil mit flachseitigem Querschnitt (71), der innerhalb des Befestigungselements (2) verschiebbar ist, wobei genannte Querschnitte (73, 71) durch eine Flanke (72) verbunden sind, die an einer Anlauffläche (27) innerhalb des Befestigungselements (2) anliegt.

3. Ein Gerät nach einem der vorhergehenden Ansprüche, **dadurch gekennzeichnet, dass** das Feststellmittel (6), nahe am inneren Ende (69) ein Sicherheitselement (66) umfasst, das in eine, am Schieberegler-Element (7) vorhandene, Auflagefläche einrastet, um zu verhindern, dass es vollständig davon abgleitet.

4. Ein Gerät nach einem der vorhergehenden Ansprüche, **dadurch gekennzeichnet, dass** die erste Öffnung (29) und die zweite Öffnung (79) eine konische Form aufweisen, um Werkzeuge mit unterschiedlichem Querschnitt aufzunehmen.

5. Ein Gerät nach Anspruch 1, **dadurch gekennzeichnet, dass** das Feststellelement (38) als "L" geformt ist, wobei der kleinste Teil der Innenfläche des "L" (38.1) so geneigt ist, dass er mit der anderen Innenfläche des "L" (38.2) einen Winkel bildet, der größer als 90° ist.

## Revendications

1. Dispositif de verrouillage rapide pour au moins un outil (9) sur au moins un élément de guidage (8) adapté pour ajuster la position dudit dispositif et au moins un outil (9) à une seule main sur l'élément de guidage (8) comprenant :
- un élément de fixation (2) pourvu d'au moins une première ouverture (29) pour le passage traversant d'au moins un outil (9) ;
- une base (3) pouvant être verrouillée de manière amovible sur l'élément de fixation (2) par des systèmes de clabots, pourvue d'une surface d'arrêt (31) faisant face à l'élément de fixation (2), ayant à son extrémité un élément de verrouillage (38) qui enclenche au moins partiellement l'élément de guidage (8) ;
- un élément en forme de crochet (4) verrouillé de manière rotative à la base (3) par une broche (43) entre un état de libération (A) et un état de verrouillage (B) pour l'élément de guidage (8) ;
- un élément curseur (7) coulissant à l'intérieur de l'élément de fixation (2) et de la base (3) et muni d'au moins une deuxième ouverture (79) et d'une cavité (74) qui retient une saillie (47) présente sur l'élément en forme de crochet (4) ;
- un système de verrouillage (6) qui retient intérieurement l'élément curseur (7) et qui est pourvu d'une extrémité intérieure (69) menant à l'intérieur de la première ouverture (29) et de la deuxième ouverture (79) et, pendant l'utilisation, vient en butée contre l'outil (9) ;
- au moins un élément élastique (39) interposé entre la base (3) et l'élément de fixation (2) qui contrecarre leur rapprochement mutuel ;
ledit dispositif supportant une condition de libération (A) dans laquelle le dispositif (1) est libéré de l'élément de guidage (8) et de l'outil (9) ;
ledit dispositif étant **caractérisé par le fait qu'**il supporte également :
- une condition verrouillée du guide (B) dans laquelle la base est verrouillée à l'élément de guidage (8) au moyen d'une première rotation des systèmes de verrouillage (6) entraînant l'élément curseur (7) qui se déplace à l'intérieur de la base (3) en engageant la saillie (47) de l'élément en crochet (4) de telle sorte que ledit élément en crochet (4) tourne autour de la broche (43) formant un angle obtus avec la surface d'arrêt (31), ce qui permet de faire varier l'angle de rotation entre la base (3) et l'élément de fixation (2) mais aussi de déplacer l'outil (9);
- une condition verrouillée en rotation (C) dans laquelle la rotation entre l'élément de fixation (2) et la base (3) est empêchée au moyen d'une seconde rotation dans le même sens que le premier système de verrouillage (6), en approchant de l'élément de fixation (2) de la base (3) jusqu'à ce qu'ils s'engagent mutuellement, permettant de déplacer l'outil (9) ;
- une condition verrouillée de l'outil dans laquelle l'outil (9) est verrouillé sur l'élément de fixation (2) au moyen d'une troisième rotation, dans le même sens que les rotations précédentes du système de verrouillage (6), en serrant le curseur (7) et l'extrémité intérieure (69) ce qui exerce sur l'outil (9) une force permettant de le bloquer.

2. Dispositif conforme à la revendication précédente, **caractérisé par le fait que** l'élément curseur (7) comporte une portion de section circulaire (73) pouvant coulisser à l'intérieur de ladite base (3) et une portion de section à faces planes (71) pouvant coulisser à l'intérieur de l'élément de fixation (2), lesdites sections (73, 71) étant reliées par un épaulement (72) qui vient en butée contre une surface de poussée (27) interne à l'élément de fixation (2).

3. Dispositif conforme aux revendications précédentes, **caractérisé par le fait que** le système de verrouillage (6) comprend, à proximité de l'extrémité intérieure (69), un élément de sécurité (66) qui engage une assise correspondante (77) présente dans l'élément curseur (7) pour empêcher le glissement complet de celui-ci.

4. Dispositif conforme aux revendications précédentes, **caractérisé par le fait que** la première ouverture (29) et la deuxième ouverture (79) présentent une forme conique pour recevoir des outils ayant des sections transversales différentes.

5. Dispositif conforme à la revendication 1, **caractérisé par le fait que** l'élément de verrouillage (38) est en forme de « L » avec la portion la plus petite de la face interne du « L » (38.1) inclinée de manière à former un angle de plus de 90° avec l'autre face interne (38.2) du « L ».
